(19) Europäisches Patentamt / European Patent Office / Office européen des brevets

(11) **EP 1 247 793 B1**

(12) **FASCICULE DE BREVET EUROPEEN**

(45) Date de publication et mention de la délivrance du brevet:
**09.01.2008 Bulletin 2008/02**

(51) Int Cl.:
*C07C 67/08* (2006.01)     *C07C 67/62* (2006.01)
*C07C 69/54* (2006.01)

(21) Numéro de dépôt: **02290718.2**

(22) Date de dépôt: **21.03.2002**

(54) **Procédé perfectionné de fabrication d'acrylate de 2-éthylhexyle**

Verbessertes Verfahren zur Herstellung von 2-Ethylhexylakrylat

Improved process for the manufacture of 2-ethylhexyl acrylate

(84) Etats contractants désignés:
**AT BE CH CY DE DK ES FI FR GB GR IE IT LI LU MC NL PT SE TR**

(30) Priorité: **02.04.2001 FR 0104435**

(43) Date de publication de la demande:
**09.10.2002 Bulletin 2002/41**

(73) Titulaire: **ARKEMA FRANCE**
**92700 Colombes (FR)**

(72) Inventeurs:
• **Bessalem, Jacqueline**
**57500 Saint-Avold (FR)**
• **Riondel, Alain**
**57600 Forbach (FR)**

(74) Mandataire: **Bonnel, Claudine et al**
**ARKEMA FRANCE**
**Département Propriété Industrielle**
**420, rue d'Estienne d'Orves**
**92705 Colombes Cedex (FR)**

(56) Documents cités:
**EP-A- 0 609 127**     **EP-A- 0 687 664**

• **CHEMICAL ABSTRACTS, vol. 90, no. 26, 25 juin 1979 (1979-06-25) Columbus, Ohio, US; abstract no. 204830, J. BERANEK: "Refining of acrylic or methacrylic acid esters" page 23; colonne 2; XP002188563 & CS 176 915 A 15 février 1979 (1979-02-15)**

**Description**

La présente invention concerne un procédé perfectionné de fabrication d'acrylate de 2-éthylhexyle par une estérification directe de l'acide acrylique par le 2-éthylhexanol, cette réaction étant catalysée par de l'acide sulfurique.

Dans ce procédé industriel, pour déplacer l'équilibre réactionnel, il n'est pas rajouté de solvant entraînant azéotropiquement l'eau de réaction, mais cette fonction est assurée par un excès de l'alcool estérifiant (en l'occurrence, le 2-éthylhexanol), qui présente la particularité de constituer un azéotrope avec l'eau.

À l'issue de l'étape réactionnelle réalisée en discontinu, la quasi-totalité de l'acide sulfurique a été transformée en sulfate acide de 2-éthylhexyle (Et2HexSO$_4$H), selon la réaction suivante d'estérification de l'acide sulfurique par le 2-éthylhexanol :

$$Et2HexOH + H_2SO_4 \longrightarrow HEt2HexSO_4 + H_2O$$

Par conséquent, le mélange réactionnel, en fin de réaction, comprend de l'acrylate de 2-éthylhexyle, du 2-éthyl-hexanol, de l'acide acrylique, du sulfate acide de 2-éthylhexyle, des traces d'acide sulfurique, et les stabilisants classiquement utilisés dans la réaction.

Après la réaction d'estérification, on neutralise les espèces acides contenues dans le mélange brut réactionnel par addition à celui-ci d'une solution aqueuse de base (soude) ; pendant cette étape, l'acide acrylique est neutralisé en acrylate de sodium, le sulfate acide de 2-éthylhexyle, en sulfate neutre de 2-éthylhexyle (Et2HexSO$_4$Na), et les traces d'acide sulfurique, en sulfate de sodium Na$_2$SO$_4$, tous ces sels passant dans la phase aqueuse. La phase organique et la phase aqueuse issues de cette neutralisation sont séparées. L'acrylate de 2-éthylhexyle recherché est récupéré par distillation à partir de la phase organique, et le 2-éthylhexanol est récupéré par distillation à partir de la phase aqueuse. Ces étapes de purification qui font suite à la réaction d'estérification sont généralement effectuées en continu.

Le document EP 687 664 décrit un tel procédé en présence de phénothiazine comme stabilisant de l'acide acrylique.

Lors de la mise en oeuvre de ce procédé de fabrication de l'acrylate de 2-éthylhexyle, il apparaît qu'il se forme une émulsion dense à l'interphase lors de la décantation qui se fait suite à l'étape de neutralisation précitée. Cette émulsion, qui pourrait être due à des polymères d'acrylate de 2-éthylhexyle se développant à l'interphase, perturbe le train de distillation en aval et abaisse les performances environnementales de l'atelier de fabrication.

Cherchant à résoudre ce problème, la Société déposante a découvert de façon surprenante que l'hydroquinone, stabilisant couramment utilisé pour l'acide acrylique, ne devait pas être utilisé et que, s'il l'était, au moins un stabilisant de phase aqueuse, spécifique, devait être ajouté afin d'éviter l'apparition de cette émulsion.

La demande internationale PCT WO 98/56746 et les brevets américains US-A-5 928 558 et 5 932 735 décrivent d'une manière générale l'utilisation du 2,2,6,6-tétraméthyl-1-pipéridinyloxy (TEMPO) et de ses dérivés comme stabilisant de l'acide (méth)acrylique en présence d'eau.

La présente invention a pour objet, dans la fabrication de l'acrylate de 2-éthylhexyle selon un procédé d'estérification directe de l'acide acrylique par le 2-éthylhexanol, catalysée par l'acide sulfurique, l'acide acrylique soumis à l'estérification étant stabilisé par au moins un agent stabilisant, ladite estérification étant suivie par la neutralisation par une base du mélange réactionnel brut (B1) obtenu, les sels résultants passant dans la phase aqueuse (A1) dudit mélange, la phase organique (O1) et la phase aqueuse (A1) issues de cette neutralisation étant séparées et l'acrylate de 2-éthylhexyle étant récupéré à partir de la phase organique (O1), un procédé de suppression des émulsions qui apparaissent lors de ladite neutralisation, caractérisé par le fait que l'on choisit pour stabilisant de l'acide acrylique l'hydroquinone, et que l'on stabilise la phase aqueuse du procédé avant et/ou pendant la neutralisation.

La présente invention a donc pour objet un procédé de fabrication d'acrylate de 2-éthylhexyle par estérification directe de l'acide acrylique par le 2-éthylhexanol, en présence d'au moins un stabilisant pour l'acide acrylique, ladite estérification étant catalysée par l'acide sulfurique, le mélange réactionnel brut obtenu comprenant de l'acrylate de 2-éthylhexyle, du 2-éthyl-hexanol, de l'acide acrylique, du sulfate acide de 2-éthyl-hexyle, des traces d'acide sulfurique et les impuretés habituelles, procédé suivant lequel on fait suivre l'estérification par l'addition audit mélange réactionnel brut d'une base pour neutraliser l'acide acrylique, le sulfate acide 2-éthylhexyle, les traces d'acide sulfurique qui y sont présents, les sels résultants passant dans la phase aqueuse (A1) dudit mélange, la phase organique (O1) et la phase aqueuse (A1) issues de cette neutralisation étant séparées et l'acrylate de 2-éthylhexyle recherché étant récupéré à partir de la phase organique (O1) ;

(1) la récupération du 2-éthylhexanol dans (A1) à partir de ladite phase aqueuse, les eaux usées (E2) débarrassées dudit alcool étant rejetées,

caractérisé par le fait le stabilisant de l'acide acrylique est l'hydroquinone, et que l'on stabilise la phase aqueuse du procédé avant ou pendant la neutralisation par l'introduction d'au moins un stabilisant de phase aqueuse choisi parmi les sels métalliques de Fe, Mn et Cu, et le 2,2,6,6-tétraméthyl-1-pipéridinyloxy (TEMPO), le 4-hydroxy-2,2,6,6-tétraméthyl-1-pipéridinyloxy (4-hydroxy TEMPO), le 4-méthoxy-2,2,6,6-tétraméthyl-1-pipéridinyloxy(4-méthoxy-TEMPO) et le 4-oxo-2,2-6,6-tétraméthyl-1-pipéridinyloxy(4-oxo-TEMPO).

Les sels métalliques que l'on peut utiliser sont notamment choisis parmi $CuSO_4$, $Fe_2(SO_4)_3$ et l'acétate de manganèse $Mn(OAc)_2$.

On peut introduire le ou les stabilisants de phase aqueuse dans le réacteur d'estérification et/ou dans le réacteur de neutralisation et/ou dans une partie de l'appareillage située entre ces deux réacteurs.

Par ailleurs, ce ou ces stabilisants de phase aqueuse sont généralement introduits à raison de 20 à 1000 pm, en particulier de 50 à 200 ppm, par rapport au brut réactionnel (B1).

Le stabilisant d'acide acrylique est généralement présent à raison de 200 à 2000 ppm par rapport à l'acide acrylique.

La purification qui fait suite à la neutralisation peut être effectuée de façon traditionnelle, par les opérations décrites dans le brevet européen EP-B-609 127.

Les Exemples suivants illustrent la présente invention sans toutefois en limiter la portée.

### EXEMPLE 1 (Référence)

On a conduit en discontinu, dans un réacteur agité, à 80□C, sous pression réduite, une estérification de l'acide acrylique par le 2-éthylhexanol, en présence d'acide sulfurique comme catalyseur, et en présence d'hydroquinone comme inhibiteur de polymérisation.

Le brut réactionnel (B1) ainsi constitué est neutralisé pour éliminer les acides présents, à savoir le sulfate acide de 2-éthylhexyle, l'acide acrylique et les traces d'acide sulfurique. Cette neutralisation a lieu dans un autre réacteur agité en discontinu à partir de 200 g de brut B1, avec une solution de soude à 4% en poids (durée 30 minutes à température ambiante). La décantation est réalisée dans une ampoule à décanter. Dans ce cas, on note l'apparition d'une émulsion à l'interphase lors de la décantation.

### EXEMPLES 2-3 (Référence) et 4 à 7 (de l'invention)

On a conduit la même réaction qu'à l'Exemple 1 avec un stabilisant d'acide acrylique et un stabilisant de phase aqueuse. Les résultats sont rapportés dans le Tableau suivant :

Tableau

| Exemple | Stabilisant de l'Acide Acrylique | Stabilisant de Phase Aqueuse | Apparition d'une émulsion à la neutralisation |
|---|---|---|---|
| 2 | EMHQ | - | NON |
| 3 | PTZ | - | NON |
| 4 | HQ | $CuSO_4$ | NON |
| 5 | HQ | $Fe_2(SO_4)_3$ | NON |
| 6 | HQ | $Mn(OAc)_2$ | NON |
| 7 | HQ | TEMPO | NON |

### Revendications

1. - Procédé de fabrication d'acrylate de 2-éthylhexyle par estérification directe de l'acide acrylique par le 2-éthylhexanol, en présence d'au moins un stabilisant pour l'acide acrylique, ladite estérification étant catalysée par l'acide sulfurique, le mélange réactionnel brut (B1) obtenu comprenant de l'acrylate de 2-éthylhexyle, du 2-éthylhexanol, de l'acide acrylique, du sulfate acide de 2-éthylhexyle, des traces d'acide sulfurique et les impuretés habituelles, procédé suivant lequel on fait suivre l'estérification par

   (a) l'addition audit mélange réactionnel brut (B1) d'une base pour neutraliser l'acide acrylique, le sulfate acide de 2-éthylhexyle, les traces d'acide sulfurique qui y sont présents, les sels résultants passant dans la phase aqueuse (A1) dudit mélange, la phase organique (O1) et la phase aqueuse (A1) issues de cette neutralisation étant séparées et l'acrylate de 2-éthylhexyle recherché étant récupéré à partir de la phase organique (O1);

(b) la récupération du 2-éthylhexanol dans (A1) à partir de ladite phase aqueuse, les eaux usées (E2) débarrassées dudit alcool étant rejetées,

**caractérisé par le fait que** le stabilisant de l'acide acrylique est l'hydroquinone et que l'on introduit dans la phase aqueuse du procédé avant/ou pendant la neutralisation au moins un stabilisant de phase aqueuse choisi parmi les sels métalliques de Fe, Mn et Cu, et le 2,2,6,6-tétraméthyl-1-pipéridinyloxy (TEMPO), le 4-hydroxy-2,2,6,6-tétraméthyl-1-pipéridinyloxy (4-hydroxy TEMPO), le 4-méthoxy-2,2,6,6-tétraméthyl-1-pipéridinyloxy(4-méthoxy-TEMPO) et le 4-oxo-2,2-6,6-tétraméthyl-9-pipéridinyloxy(4-oxo-TEMPO).

**2.** - Procédé selon la revendication 1, **caractérisé par le fait que** l'on choisit les sels métalliques parmi $CuSO_4$, $Fe_2(SO_4)_3$ et l'acétate de manganèse $Mn(OAc)_2$.

**3.** - Procédé selon la revendication 1 ou 2, **caractérisé par le fait que** l'on introduit le ou les stabilisants de phase aqueuse dans le réacteur d'estérification et/ou dans le réacteur de neutralisation et/ou dans une partie de l'appareillage située entre ces deux réacteurs.

**4.** - Procédé selon l'une des revendications 1 à 3, **caractérisé par le fait que** l'on introduit le ou les stabilisants de phase aqueuse à raison de 20 à 1000 ppm par rapport au brut réactionnel (B1).

**5.** - Procédé selon l'une des revendications 1 à 4, **caractérisé par le fait que** le stabilisant d'acide acrylique est présent à raison de 200 à 2000 ppm par rapport à l'acide acrylique.

**Claims**

**1.** Process for the manufacture of 2-ethylhexyl acrylate by direct esterification of acrylic acid by 2-ethylhexanol in the presence of at least one stabilizer for acrylic acid, the said esterification being catalysed by sulphuric acid and the crude reaction mixture (B1) obtained comprising 2-ethylhexyl acrylate, 2-ethylhexanol, acrylic acid, 2-ethylhexyl hydrogen sulphate, traces of sulphuric acid and the usual impurities, following which process the esterification is followed by

(a) the addition to the said crude reaction mixture (B1) of a base in order to neutralize the acrylic acid, 2-ethylhexyl hydrogen sulphate and the traces of sulphuric acid which are present therein, the resulting salts passing into the aqueous phase (A1) of the said mixture, the organic phase (O1) and the aqueous phase (A1) resulting from this neutralization being separated and the desired 2-ethylhexyl acrylate being recovered from the organic phase (O1);
(b) the recovery of the 2-ethylhexanol in (A1) from the said aqueous phase, the aqueous effluents (E2) freed from the said alcohol being discharged,

**characterized in that** the stabilizer for acrylic acid is hydroquinone and **in that** at least one aqueous phase stabilizer chosen from Fe, Mn and Cu metal salts and 2,2,6,6-tetramethyl-1-piperidinyloxy (TEMPO), 4-hydroxy-2,2,6,6-tetramethyl-1-piperidinyloxy (4-hydroxy-TEMPO), 4-methoxy-2,2,6,6-tetramethyl-1-piperidinyloxy (4-methoxy-TEMPO) and 4-oxo-2,2,6,6-tetramethyl-1-piperidinyloxy (4-oxo-TEMPO) is introduced into the aqueous phase of the process before or during the neutralization.

**2.** Process according to Claim 1, **characterized in that** the metal salts are chosen from $CuSO_4$, $Fe_2(SO_4)_3$ and manganese acetate $Mn(OAc)_2$.

**3.** Process according to Claim 1 or 2, **characterized in that** the aqueous phase stabilizer or stabilizers is/are introduced into the esterification reactor and/or into the neutralization reactor and/or into a part of the equipment situated between these two reactors.

**4.** Process according to one of Claims 1 to 3, **characterized in that** the aqueous phase stabilizer or stabilizers is/are introduced in a proportion of 20 to 1000 ppm, with respect to the crude reaction product (B1).

**5.** Process according to one of Claims 1 to 4, **characterized in that** the acrylic acid stabilizer is present in a proportion of 200 to 2000 ppm, with respect to the acrylic acid.

**Patentansprüche**

1. Verfahren zur Herstellung von 2-Ethylhexylacrylat durch Direktveresterung von Acrylsäure mit 2-Ethylhexanol in Gegenwart mindestens eines Stabilisators für Acrylsäure, wobei die Veresterung durch Schwefelsäure katalysiert wird und die erhaltene rohe Reaktionsmischung (B1) 2-Ethylhexylacrylat, 2-Ethylhexanol, Acrylsäure, 2-Ethylhexylhydrogensulfat, Spuren von Schwefelsäure und die üblichen Verunreinigungen enthält, gemäß dem man nach der Veresterung

   (a) die rohe Reaktionsmischung (B1) zur Neutralisierung der Acrylsäure, des 2-Ethylhexylhydrogensulfats und der Spuren von Schwefelsäure, die darin vorliegen, mit einer Base versetzt, wobei die erhaltenen Salze in die wäßrige Phase (A1) der Mischung übergehen, die organische Phase (O1) und die wäßrige Phase (A1) aus dieser Neutralisation getrennt werden und das gewünschte 2-Ethylhexylacrylat aus der organischen Phase (O1) gewonnen wird;
   (b) das 2-Ethylhexanol in (A1) aus der wäßrigen Phase gewinnt, wobei die von dem Alkohol befreiten Abwässer (E2) verworfen werden,

   **dadurch gekennzeichnet, daß** es sich bei dem Acrylsäure-Stabilisator um Hydrochinon handelt und man vor oder während der Neutralisation mindestens einen unter Metallsalzen von Fe, Mn und Cu, 2,2,6,6-Tetramethyl-1-piperidinyloxy (TEMPO), 4-Hydroxy-2,2,6,6-tetramethyl-1-piperidinyloxy (4-Hydroxy-TEMPO), 4-Methoxy-2,2,6,6-tetramethyl-1-piperidinyloxy (4-Methoxy-TEMPO) und 4-Oxo-2,2,6,6-tetramethyl-1-piperidinyloxy (4-Oxo-TEMPO), ausgewählten Stabilisator der wäßrigen Phase in die wäßrige Phase des Verfahrens einträgt.

2. Verfahren nach Anspruch 1, **dadurch gekennzeichnet, daß** man die Metallsalze unter $CuSO_4$, $Fe_2(SO_4)_3$ und Manganacetat $Mn(OAc)_2$ auswählt.

3. Verfahren nach Anspruch 1 oder 2, **dadurch gekennzeichnet, daß** man den Stabilisator bzw. die Stabilisatoren der wäßrigen Phase in den Veresterungsreaktor und/oder in den Neutralisationsreaktor und/oder in einen Teil der zwischen diesen beiden Reaktoren liegenden Apparatur einträgt.

4. Verfahren nach einem der Ansprüche 1 bis 3, **dadurch gekennzeichnet, daß** man den Stabilisator bzw. die Stabilisatoren der wäßrigen Phase in einer Menge von 20 bis 1000 ppm, bezogen auf die rohe Reaktionsmischung (B1), einträgt.

5. Verfahren nach einem der Ansprüche 1 bis 4, **dadurch gekennzeichnet, daß** der Acrylsäure-Stabilisator in einer Menge von 200 bis 2000 ppm, bezogen auf die Acrylsäure, vorliegt.

**RÉFÉRENCES CITÉES DANS LA DESCRIPTION**

**Documents brevets cités dans la description**

- EP 687664 A
- WO 9856746 A
- US 5928558 A
- US 5932735 A
- EP 609127 B